# EUROPÄISCHE PATENTSCHRIFT

(11) **EP 3 804 806 B1**
(45) Veröffentlichungstag und Bekanntmachung des Hinweises auf die Patenterteilung: **14.02.2024**
(21) Anmeldenummer: 20198639.5
(22) Anmeldetag: 28.09.2020
(51) Int. Cl.: A61N 1/05, A61N 1/37, A61N 1/44

(54) **IMPLANTIERBARE ELEKTRODENANORDNUNG UND HERSTELLUNGSVERFAHREN**
IMPLANTABLE ELECTRODE ASSEMBLY AND METHOD OF MANUFACTURING THE SAME
DISPOSITIF D'ÉLECTRODE IMPLANTABLE ET PROCÉDÉ DE FABRICATION

(30) Priorität: 11.10.2019 DE 102019215673
(43) Veröffentlichungstag der Anmeldung: 14.04.2021
(73) Patentinhaber: Albert-Ludwigs-Universität Freiburg, 79098 Freiburg (DE)
(72) Erfinder: SCHULTE, Jennifer, 79106 Freiburg (DE); STIEGLITZ, Thomas, 79110 Freiburg (DE); EICKENSCHEIDT, Max, 79111 Freiburg (DE)
(74) Vertreter: Grünecker Patent- und Rechtsanwälte PartG mbB

(56) Entgegenhaltungen:
- DE-A1- 4 207 368
- DE-A1- 19 927 615
- US-A1- 2015 148 879
- US-A1- 2019 030 318

## Beschreibung

Die vorliegende Erfindung bezieht sich auf vorzugsweise flexible implantierbare Elektrodenanordnungen, z. B. Elektrodenarrays, und auf ein zugehöriges Entwurfsverfahren. Insbesondere befasst sich die vorliegende Erfindung mit dem Entwurf von Elektrodenanordnungen mit Dünnfilmmetallisierung.

Jüngste Forschung und Entwicklung auf dem Gebiet der Neurotechnik ("Neural Engineering") führten zu einer Vielzahl von aktiven implantierbaren medizinischen Geräten (Active Implantable Medical Device, AIMD), die in einem weiten Anwendungsbereich einsetzbar sind. Diese bestehen üblicherweise aus einem Gehäuse, das eine Steuerelektronik und eine Batterie beinhaltet, implantierbaren Elektroden (oder Elektrodenarrays) und Kabeln für die elektrische Kontaktierung der Elektroden und der Elektronik. Die Elektroden werden für die elektrische Stimulierung von Zellen oder das Erfassen von physiologischen Signalen verwendet.

Damit dienen neuronale Elektroden als Schnittstelle zwischen dem biologischen und dem technischen System, wobei ihre Aufgabe im Wesentlichen in der Aufzeichnung und/oder der Anregung neuronaler Signale besteht. Wenn neuronale Elektroden in AIMD eingesetzt werden, spielen sie eine Schlüsselrolle bei der Wiederherstellung und Aufrechterhaltung von Körperfunktionen bei Patienten mit physischen Einschränkungen und auch psychiatrischen Erkrankungen. Derartige Elektroden besitzen ein elektrisch leitfähiges Material für die Kontaktbereiche und die Verbindungsstellen sowie ein Substratmaterial, das die elektrisch leitfähigen Materialien isoliert. Wesentliche Voraussetzungen für den Erfolg implantierbarer Medizingeräte ist zum einen eine vorteilhafte Gewebe-Elektroden-Wechselwirkung und zum anderen eine ausreichende Biostabilität. Aus diesem Grund ist die mechanische Flexibilität der Elektrode ein essenzieller Aspekt beim Design von neuronalen Sonden, um eine strukturelle Biokompatibilität zu erzielen und dadurch die Fremdkörperreaktion zu verringern und die Lebensdauer des Implantats zu erhöhen.

Insbesondere werden implantierbare Systeme eingesetzt, welche Elektrodenmetallisierungen basierend auf Dünnfilm-Technologie verwenden, um durch gezielte, spezifische elektrische Interaktion mit einzelnen Neuronen im Nervensystem ausgefallene Körper- und Sinnesfunktionen zu unterstützen oder wiederherzustellen, ebenso wie Funktionsstörungen von Organen zu beheben. Um die dafür hohe räumliche Auflösung zu erreichen, werden die Elektrodenmetallisierungen als dünne Schichten auf einem Trägersubstrat mit Durchmessern im Mikrometerbereich und Schichtdicken von nur wenigen Hundert Nanometern realisiert. Durch die Miniaturisierung kommt es zu einem Verlust der chemischen, physikalischen und mechanischen Stabilität der Metalle bei Delamination und somit zum frühzeitigen Funktionsausfall, da die Dünnfilme Dicken im Bereich oder unterhalb einzelner Körner im Gefüge aufweisen.

Bisherige auf Dünnfilmtechnologie basierende Elektroden leiden unter Degradationsmechanismen wie Korrosion, Rissbildung und Verlust der Haftung und somit Delamination des Dünnfilms vom Trägersubstrat in Folge von chemischer und physikalischer Materialermüdung und Freisetzen von Dehnungsenergie.

Die meisten herkömmlichen Dünnschichtelektroden verwenden mittels Dünnfilmtechnologie aufgebrachtes Platin oder Platin mit einem auf das Platin gesputterten Iridium/Iridiumoxidfilm mit oder ohne Haftvermittlerschichten zum Trägersubstrat als Metallisierungsschicht.

Durch die zyklisch alternierenden Oxidations- und Reduktionsreaktionen der Metallisierungsoberfläche bei elektrischer Stimulation kommt es zur wechselnden chemischen Materialänderung durch Oxidation, Chloridkomplexbildung, Wasserstoff-Versprödung oder Volumenausdehnung durch Wasserstoffeinlagerung und resultierender mechanischer Spannungsausbreitung (Zug- und Druckspannung) im Mikrogefüge, wodurch Ermüdungs- und Verformungsprozesse wie plastische Verformung, Rissbildung und Haftungsverlust der Dünnschichten und Grenzflächen ausgelöst werden.

Durch die während der elektrischen Stimulation meist nicht chemisch ausgeglichenen Reaktionsraten und die durch den elektrischen Stromfluss verstärkte Korrosion kommt es zu einer Beschleunigung der Deformationsmechanismen.

Beispielsweise befasst sich der Artikel J. Pfau, T. Stieglitz, and J. Ordonez, "Mechanical deformation and chemical degradation of thin-film platinum under aging and electrical stimulation," in 2017 8th International IEEE/EMBS Conference on Neural Engineering (NER): 25-28 May 2017, Shanghai, China, 2017, pp. 166-169, mit Alterungs- und Degradierungsprozessen implantierbarer Elektroden. Das Dokument beschreibt den Einfluss der elektrischen Stimulation auf die Alterung der Elektroden. Insbesondere wird der wechselnde Einbau und Ausstoß von Sauerstoff und Wasserstoff als Ursache für die Deformation gesehen. Weiterhin beschreibt der Artikel J. S. Ordonez, L. Rudmann, P. Cvancara, C. Bentler, and T. Stieglitz, "Mechanical deformation of thin film platinum under electrical stimulation", Conference proceedings: Annual International Conference of the IEEE Engineering in Medicine and Biology Society. IEEE Engineering in Medicine and Biology Society. Annual Conference, vol. 2015, pp. 1045-1048, 2015, das Auftreten von mechanischer Deformation infolge der elektrischen Stimulation von Platin-Dünnfilmelektroden.

US-A1-2019/030318 offenbart Mikroelektroden mit fraktaler Geometrie. Die Druckschrift offenbart insbesondere ein Elektrodendesign zur Verlängerung der Lebensdauer und Funktionseffizienz von implantierbaren Impulsgeneratoren. Die vorgeschlagene Elektrode liefert angeblich effizienter elektrische Ladung zur Stimulierung des Nervensystems, reduziert den Stromverbrauch um bis zu 50 Prozent und erhöht gleichzeitig die Effektivität der Funktionalität. Das offenbarte Elektrodendesign kann in implantierbaren Simulationssystemen verwendet werden, um eine große Anzahl neurologischer Störungen mit bestehenden Plattformen zu behandeln oder eigenständig durchzuführen.

DE-A1-199 27 615 offenbart eine implantierbare Detektionsvorrichtung zur Erkennung von Ischämie-Zuständen des Herzens, die versehen ist mit einer Stimulationseinrichtung zur Abgabe eines Stimulationsimpulses an das Herz, einer Elektroden-Sensoranordnung zur Detektion des durch einen Stimulationsimpuls erzeugten, ventrikulär evozierten Reizantwort-Signals des Herzens, einer Blutströmungs-Sensoranordnung zur indirekten oder direkten Erfassung des Blutversorgungszustandes des Myocards und einer Erfassungseinrichtung zur Auswertung und/oder Weitergabe der von den Sensoranordnungen gelieferten Signale.

DE-A1-42 07 368 betrifft ebenfalls eine Herzschrittmacherelektrode. Insbesondere ist eine Stimulationselektrode offenbart, die insbesondere aus Titan besteht und mit einer porösen Oberflächenbeschichtung versehen ist, deren aktive Oberfläche größer ist als die sich aus der geometrischen Form der Elektrode ergebende Oberfläche, wobei die aktive Oberfläche durch eine, insbesondere fraktale, räumliche Geometrie um einen Faktor von mindestens tausend größer ist als die sich aus der geometrischen Form der Elektrode ergebende Oberfläche und die Oberflächenbeschichtung aus einem inerten Material besteht.

US-A1-2015/148879 offenbart eine implantierbare Elektrodenanordnung für kardiologische Geräte wie Herzschrittmacher, die einen länglichen Elektrodenkörper mit einem proximalen Ende und einem distalen Ende umfasst, und mindestens eine Elektrode, die den Körper des Implantatträgers berührt und an oder in der Nähe des distalen Endes des Elektrodenkörpers angeordnet ist. Die implantierbare Elektrodenanordnung umfasst mindestens eine die Elektrode kontaktierende elektrische Kontaktleitung und eine mit der Elektrode oder einer Kontaktleitung verbundene elektromechanische Resonanzanordnung zur Umwandlung von in die Elektrodenleitung eingespeisten Hochfrequenzsignalen in akusto-mechanische Schwingungen.

Um die Funktionalität der Elektroden über den gesamten Zeitraum der Intervention mit dem Nervensystem zu gewährleisten, ist es erforderlich, die auftretenden Fehlermechanismen zu kompensieren oder möglichst zu beheben. Es besteht daher ein Bedarf an einem Verfahren zum Entwerfen flexibler implantierbarer Elektrodenanordnungen, welches die Nachteile der bekannten Lösungen überwindet, so dass die gefertigten Elektrodenanordnungen sicher und zuverlässig sind, aber dennoch kostengünstig sowie auf Standardtechnologiebasis herstellbar sind. Weiterhin besteht ein Bedarf an einer derartigen flexiblen implantierbaren Elektrodenanordnung.

Diese Aufgabe wird durch den Gegenstand der unabhängigen Patentansprüche gelöst. Vorteilhafte Ausführungsformen der vorliegenden Erfindung sind Gegenstand der abhängigen Patentansprüche.

Dabei basiert die vorliegende Erfindung auf der Idee, Elektroden entsprechend ihrer mechanischen Schwingungsantwort auf die einwirkenden elektrischen Feldparameter zur Stimulation auf eine an die Stimulationsparameter adaptierte Schwingungskompensation und ein Verformungsminimum hin zu entwerfen. Bisher wurden Elektroden noch nie als elektrisch stimuliertes, schwingfähiges System (Oszillator) angesehen und daher nie auf eine mechanische Schwingungsantwort auf die elektrochemische Stimulation untersucht, was zur Folge hat, dass bisherige Elektrodendesignansätze die elektrochemisch-mechanische Spannungskopplung vollständig außer Acht lassen und durch fehlende Spannungskompensation eine für die chronische Implantation und elektrische Stimulation nicht ausreichende chemische und mechanische Stabilität aufweisen.

Die Erfinder der vorliegenden Erfindung haben erkannt, dass eine Optimierung der Elektroden im Hinblick auf eine möglichst geringe Resonanz in Antwort auf Anregungsspannungen mit für die in der elektrischen Stimulation von Nerven wichtigen Frequenzen (im Bereich von 1 Hz- 100 kHz, vorzugsweise zwischen 1 Hz und 10 kHz sowie zwischen 20 kHz und 40 kHz) und Pulsformen dazu führt, dass die oben beschriebenen Ausfall- und Degradationsmechanismen gar nicht oder nur in abgemilderter Form auftreten.

Die Dünnfilmelektroden werden durch lokale Änderungen und Anpassungen der geometrischen Form und Strukturierung der Oberflächentopografie sowie der Haftvermittlerschichttopografie auf ein globales Schwingungsminimum für die zur elektrischen Stimulation von Nerven wichtigen Frequenzen im Bereich von 1 Hz- 100 kHz und Pulsformen entworfen. Die Designansätze werden so angepasst, dass eine lokale Anpassung der mechanischen Schwingung durch lokale mechanische Verstärkung oder Schwächung der Elektrodenstruktur und/oder -topografie für eine Reduktion der globalen mechanischen Resonanz (in Schwingungsamplitude und Eigenschwingungsfrequenzverhalten) der Elektrode erfolgt.

Vorzugsweise wird dabei als Randbedingung der äußere Elektrodenausschnitt, welcher durch das Trägersubstrat offengelegt ist, so gewählt, dass er die gleiche globale Fläche der Elektrode auf dem Trägersubstrat einnimmt wie eine Standardelektrode mit kreisförmiger Öffnung. Die Designstrukturierung erfolgt so, dass die mechanische Schwingung und der Resonanzfall als Antwort auf das angelegte elektrische Feld reduziert wird. Die Änderungen des Elektrodenentwurfs bezieht sich auf lokale (nicht globale) Anpassungen der Elektrodengeometrie und Topografie. Dabei wird vorzugsweise von Dünnfilmen mit Schichtdicken im Bereich von 10 nm bis zu 2 µm ausgegangen. Eine zugehörige Haftvermittlerschicht hat eine Dicke im Bereich zwischen 10 nm und 50 nm.

Insbesondere umfasst eine (vorzugsweise flexible) implantierbare Elektrodenanordnung eine elektrisch isolierende Trägerstruktur und eine elektrisch leitfähige Schicht, die eine elektrisch leitfähige Dünnfilmschicht aufweist, wobei die elektrisch leitfähige Dünnfilmschicht strukturiert ist, um wenigstens eine implantierbare Elektrode auszubilden. Erfindungsgemäß weist die wenigstens eine Elektrode eine lokale Selbstähnlichkeit (Fraktalisierung) auf, die so gewählt ist, dass eine mechanische Resonanz der Elektrode auf eine elektrische Anregung mit einem Anregungssignal, insbesondere einer Anregungsspannung oder einem Anregungsstrom, ein Minimum annimmt. Insbesondere weist die Elektrode eine Selbstähnlichkeit der Rand- oder Grundfläche oder der Topografie auf, wobei die Geometrie eine gebrochene Dimension, d.h. eine Dimension einer nicht ganzzahligen reellen Zahl, aufweisen kann. Beispielsweise kann die Elektrode eine spiralförmige Linie aufweisen, die eine Kreisfläche ergibt.

Erfindungsgemäß wird die elektrische Feldverteilung auf die gewünschte mechanische Schwingungsdeformation der Dünnfilmelektrode optimiert. Dabei wird vorzugsweise lokal die auftretende elektrische Feldverteilung und somit die zugehörige mechanische Schwingung geändert. Damit wird eine globale Schwingungsminimierung erreicht. Beispielsweise kann dies durch verschiedene Ansätze der Selbstähnlichkeit der Randfläche, der lokalen Dünnfilmgeometrie, Dünnfilmtopografie, Haftvermittlertopografie zwischen Dünnfilm und Trägersubstrat und Dämpfung durch selbstähnliche Strukturen auf dem Substrat, welches auf der Dünnfilmoberfläche aufliegt, erreicht werden. Bei allen Ansätzen wird von Dünnfilmen ausgegangen, wobei die globale Elektrodengeometrie und die Elektrodenausschnitte im Substrat im Vergleich zu bekannten Anordnungen unverändert bleiben, um auf die Dimensionen herkömmlich eingesetzter Dünnfilmelektroden zu passen.

Die Dünnschichtsysteme berücksichtigen die während der elektrischen Stimulation *in vivo* auftretenden chemischen und mechanischen Spannungsmechanismen, die zu einer plastischen Deformation und Verlust der Schichtintegrität der Dünnschicht führen und wirken der mechanischen Deformation entgegen.

Durch den an die Resonanz angepassten Entwurf der Elektroden erfolgt eine adaptive Schwingungskompensation an die unterschiedlichen Spezifikationen der unterschiedlichen Anwendungsbereiche der funktionalen neuralen, elektrischen Stimulation, was zu einer Verbesserung der mechanischen Stabilität und Anpassung des Entwurfs an individuelle Stimulationsmuster der Dünnschichten führt und somit die Langlebigkeit der Elektroden erhöht.

Die Dünnschichtsysteme werden für eine optimale mechanische Spannungskompensation ausgelegt.

Die Elektrodenanordnung gemäß der vorliegenden Erfindung hat den Vorteil, dass Spannungsrisskorrosion, Korrosionsermüdung und Dünnschicht-Dünnschicht sowie Dünnschicht-Substrat Haftungsverlust verringert oder verhindert werden können. Es kann eine Homogenisierung des globalen elektrischen Feldes und der erzeugten lonenströme erreicht werden. Weiterhin können in vorteilhafter Weise die Schwingungseigenschaften an die anwendungsspezifischen elektrischen Stimulationsanforderungen angepasst werden.

Daraus resultierend können Degradationsmechanismen verringert oder völlig eliminiert werden und die Dünnfilmmetallisierung hat eine längere Langzeitstabilität, so dass sie besser für die Implantation und chronische Innervation mit dem Nervensystem geeignet ist. Darüber hinaus kann die Elektrodenanordnung allein basierend auf Dünnfilm-Technologie gefertigt werden.

Gemäß einer vorteilhaften Ausgestaltung der vorliegenden Erfindung umfasst die lokale Fraktalisierung eine Strukturierung eines Umrisses der Elektrode, so dass eine Elektrodenfläche beispielsweise annähernd durch eine selbstähnliche Mandelbrotgeometrie gebildet ist. Weitere Möglichkeiten zur Ausgestaltung sind Pythagoras-Baum-Fraktale oder Levysche Kurven.

Das Wort Fraktal wurde von Benoit B. Mandelbrot geprägt und ist aus dem lateinischen "frangere" abgeleitet, was so viel wie gebrochen, zerstückelt oder uneben heißt. Das Wort Fraktal wird aus einer Eigenschaft der angesprochenen Formen abgeleitet, nämlich der im Gegensatz zur topologischen Dimension gebrochenen Zahl, die den Zusammenhang zwischen linearer Ausdehnung und Flächeninhalt (oder Volumen) eines Gebildes beschreibt. Die wichtigsten Eigenschaften eines Fraktals sind seine fraktale Dimension (im folgenden auch Fraktalitätsfaktor genannt) und die sogenannte Selbstähnlichkeit. Durch die gebrochene Kante eines Fraktals kann man diesem keine ganzzahlige Dimension zuordnen, wie z.B. einer geraden Linie mit der Dimension 1, einem Rechteck mit der Dimension 2 oder einem 3-dimensionalen Würfel. Vielmehr liegen die Dimensionen eines Fraktals zwischen den ganzen Zahlen. Fraktale Formen sind außerdem selbstähnlich, d.h. die Gesamtstruktur eines Fraktals ist aus kleineren Strukturen zusammengesetzt, die die gleiche Form aufweisen wie die Gesamtstruktur. Eine Figur wird selbstähnlich genannt, wenn Teile der Figur kleine Kopien der ganzen Figur sind. Eine Figur ist exakt selbstähnlich, wenn sie in Teile, die exakte Kopien der ganzen Figur sind, zerlegt werden kann. Jeder beliebige Teil enthält eine exakte Kopie der ganzen Figur.

Im Zusammenhang mit der vorliegenden Erfindung soll der Begriff "Fraktalisierung" bedeuten, dass die Struktur der Elektrode wenigstens bereichsweise in ihrem Umriss, ihrer Dicke, ihrer Verbindung zum Untergrund oder ihrer Oberfläche durch Diskontinuitäten unterbrochen ist. Diese Diskontinuitäten führen dazu, dass sich das mechanische Resonanzverhalten der Elektrode auf die Anregung durch eine Wechselspannung oder elektrische Pulsfolge verändert.

Gemäß einem vorteilhaften Beispiel erfolgt eine lokale Fraktalisierung des Elektrodenrands durch selbstähnliche Geometrien durch Mandelbrot-Fraktale und Anpassen des Fraktalitätsfaktors zur Homogenisierung der elektrischen Feldverteilung über die gesamte Elektrodenfläche, wodurch die Änderung der lokalen mechanisch-elektrischen Kopplung lokal optimiert wird, während global eine mechanische Schwingungsminimierung erfolgt. Damit wird durch die geometrische Form der Elektrode das angestrebte Resonanzverhalten erzielt.

Gemäß einer weiteren vorteilhaften Ausführungsform der vorliegenden Erfindung umfasst die lokale Fraktalisierung eine Strukturierung des Umrisses der Elektrode, so dass lokal unterschiedliche Verhältnisse zwischen einem Elektrodendurchmesser und der Elektrodenfläche eingestellt sind. Beispielsweise kann der Elektrodenumriss eine sternförmige selbstähnliche Geometrie, vergleichbar mit dem fraktalen Objekt einer Koch-Kurve, haben. Für die Elektrodenstruktur gemäß der vorliegenden Erfindung relevante Beispiele sind fraktale Linienobjekte, die sich durch ihre Selbstähnlichkeit zu einem flächenfüllenden Objekt (Flächenobjekt) erschließen, insbesondere Fraktale der Koch-Kurve, Hilbert-Kurve, Gosper-Kurve, Lévy-C-Kurve, Peano-Kurve etc.

Weiterhin kann die lokale Fraktalisierung auch eine Unterbrechung der nach außen wirksamen elektrisch leitfähigen Schicht umfassen.

Insbesondere kann vorgesehen sein, dass ein lokales Fläche-zu-Rand-Verhältnis überall auf der Elektrode konstant gehalten wird, so dass auch im Zentrum der Elektrode ein Randeffekt entsteht. Dies erfolgt beispielsweise durch Ausfüllen eines vorgegebenen Elektrodenumrisses mit Linienstrukturen wie Spiralen und/oder einer Hilbert- oder Gosper-Kurve. Damit wird ein gleicher Randeffekt des auf dem Elektrodenausschnitt anliegenden elektrischen Feldes überall auf der Elektrode geschaffen, auch im Zentrum. Insbesondere werden die lokalen Dimensionen/Relationen der Substratöffnung-zu-Substrat so gewählt, dass sich ein lokaler Mikroelektrodeneffekt mit sich überlagernden elektrischen Feldern und somit überlagernden lonenströmen ergibt. Dabei sollen sich diese lokalen elektrischen Feldkomponenten wieder zu einem globalen homogenen elektrischen Feld in Bezug auf den Elektrodenausschnitt im Substrat (gemeint ist hier der äußere Durchmesser/Öffnung/Grundgeometrie der Elektrode) überlagern. Mit der Fraktalisierung der Elektrodenfläche wird die lokale Änderung der mechanischen Schwingungseigenschaften bei gleichbleibendem globalen Wirkungsradius der Elektrode zur Anpassung der lokalen mechanischen Schwingung und globalen Resonanzminimierung erzielt. Mit anderen Worten, die geometrische Fläche wird so angepasst, dass das angestrebte Resonanzverhalten erreicht wird.

Eine weitere Optimierung erfolgt durch die Vergrößerung des Umfang-zu-Fläche-Verhältnisses durch Fraktalisierung des Randes und die dadurch erzielte Vergrößerung des Elektrodenumfangs bei gleichbleibender Elektrodenfläche.

Dabei kann die Unterbrechung durch teilweises Abdecken der elektrisch leitfähigen Schicht mit einer elektrisch isolierenden Schicht oder durch Einbetten elektrisch leitfähiger Bahnen in die elektrisch isolierende Trägerstruktur ausgebildet sein. Im ersten Fall kann eine lokale mechanische Schwingungsdämpfung von der Oberseite aus erzielt werden, während im zweiten Fall direkt die lokalen lonenströme beeinflusst werden.

Gemäß einer weiteren vorteilhaften Ausführungsform der Elektrodenanordnung ist zwischen der elektrisch isolierenden Trägerstruktur und der elektrisch leitfähigen Schicht eine Haftvermittlerschicht angeordnet und die lokale Fraktalisierung umfasst eine Strukturierung der Haftvermittlerschicht, so dass die Haftung über die Elektrodenfläche verteilt variiert.

Eine solche Fraktalisierung der Elektrodenhaftvermittlerschicht zwischen Oberflächenmetallisierung und Substrat führt zur lokalen Versteifung und Setzen lokaler Ankerpunkte (nachfolgend auch "Bumps" genannt) zwischen Metallisierung und Substrat als lokale Adhäsionspromoter an den lokalen Stellen der mechanischen Schwingungsextrema der Dünnfilmmetallisierung zur lokalen Verstärkung (Adhäsion) und Nicht-adhäsion durch Nichtsetzen von Bumps an lokalen Stellen auftretender Schwingungsminima zur lokalen Schwächung (keine Adhäsion) des Dünnfilms (der Metallisierung).

Gemäß einer vorteilhaften Weiterbildung der vorliegenden Erfindung weist die lokale Fraktalisierung eine über die Elektrodenfläche verteilte Variation der Dicke der Dünnfilmschicht auf. Beispielsweise kann das Resonanzverhalten durch Oberflächenfraktalisierung oder selbstähnliche Oberflächen der Dünnfilmelektrode optimiert werden. Insbesondere kann die Elektrodenoberfläche mit einer Vielzahl von Erhebungen, die über die Elektrodenfläche verteilt sind, lokal verstärkt sein. Diese Oberflächenfraktalisierung kann z. B. durch 3D-Strukturierung mit global inhomogen verteilter, an den lokalen Stellen mechanischer Schwingungsmaxima platzierter Mikrohügelstrukturen, Bumps, zur globalen Schwingungsdämpfung/Resonanzminimierung erfolgen.

Weiterhin kann die lokale Fraktalisierung eine über die Elektrodenfläche verteilte Variation einer Defektdichte in dem elektrisch leitfähigen Material der Elektrode umfassen. Durch das Anpassen der Defektdichte wird der intrinsische Schichtstress im Dünnfilmmikrogefüge angepasst, so dass das Resonanzverhalten optimiert wird. Die Defektdichte kann z. B. durch Anpassen der Fertigungsparameter von gesputterten und aufgedampften Dünnfilmschichten gesteuert werden, sodass ein Resonanzverhalten minimiert wird.

Gemäß einer vorteilhaften Weiterbildung der vorliegenden Erfindung weist die elektrisch isolierende Trägerstruktur ein Polymermaterial auf. Für das e Polymermaterial kommen verschiedenste Kunststoffe in Frage. Beispielsweise umfassen das Polymermaterial Polyimid, PI, Polyethylenterephthalat, PET, Polyethylen, PE, Polycabonat, PC, Polyvinylchlorid, PVC, Polyamid, PA, Polytetrafluorethylen, PTFE, Polymethylmetacrylat, PMMA, Polyetheretherketon, PEEK, Polysulfon, PSU, Poly(p-xylylene), Polydimethylsiloxan, PDMS, und/oder Polypropylen, PP. Dabei können die Trägerstruktur und eine zusätzliche Deckschicht aus demselben Material oder aus unterschiedlichen Materialien hergestellt sein. Polyimid hat dabei mehrere Vorteile: Zum einen ist es im vollständig vernetzten Zustand besonders inert und chemisch stabil. Zum anderen kann es in Form einer flüssigen Vorstufe aufgeschleudert werden und weist darüber hinaus eine zweite, feste, aber noch nicht komplett ausgehärtete Vorform auf, in der z. B. die Anhaftung nachfolgender Schichten verbessert ist. Schließlich gibt es fotostruktierbare Polyimidharzsysteme, die z. B. für die Herstellung einer Deckschicht auf einfache Weise die Öffnung der Kontaktflecken ermöglichen.

Die Dünnfilmschicht der erfindungsgemäßen Elektrodenanordnung kann eine Metallisierung aus z. B. Platin, Iridium, Platin-Iridium und/oder Iridiumoxid, aber auch Tantal, Gold oder jedes andere elektrisch leitfähige Material aufweisen. Platin hat dabei den Vorteil einer sehr hohen chemischen Langzeitstabilität. Weiterhin können die Dünnfilmmetallschichten mit anderen Metallen wie rauhem Platin, Iridiumoxid, aber auch mit kohlenstoffhaltigen Materialien (z. B. Graphen, glasartigem Kohlenstoff, Laser induziertem Kohlenstoff) beschichtet sein.

Die vorliegende Erfindung bezieht sich außerdem auf ein Verfahren zum Entwerfen einer Elektrodenanordnung, wobei das Verfahren die folgenden Schritte umfasst:
Bereitstellen einer Startkonfiguration einer flexiblen implantierbaren Elektrodenanordnung mit einer elektrisch isolierenden Trägerstruktur und einer elektrisch leitfähigen Schicht, die eine elektrisch leitfähige Dünnfilmschicht aufweist, wobei die elektrisch leitfähige Dünnfilmschicht strukturiert ist, um wenigstens eine implantierbare Elektrode auszubilden;
Anregen der Elektrodenanordnung mit einer alternierenden Anregungsspannung oder mit einem alternierendem Anregungsstrom;
Ermitteln einer mechanischen Auslenkung der Elektrodenanordnung in Antwort auf die Anregung mit der Anregungsspannung oder dem Anregungsstrom;
Modifizieren der Elektrodenanordnung, so dass eine mechanische Resonanz der Elektrode auf die elektrische Anregung mit der Anregungsspannung oder der Anregungsstrom ein Minimum annimmt.

In vorteilhafter Weise umfasst das Modifizieren der Elektrodenanordnung eine lokale Fraktalisierung der Elektrode in einer der oben erläuterten Ausführungsformen.

Es werden nachfolgend die folgenden Begriffe und Definitionen verwendet.

Der Begriff "Fraktalisierung" umfasst jede Art von Diskontinuität in der Fläche oder in der räumlichen Struktur einer Elektrode, die Einfluss auf das Resonanzverhalten der Elektrode hat.

Der Begriff "Dünnfilmelektrode" bezeichnet eine elektrisch leitfähige Schicht mit Schichtdicken in einem Bereich zwischen 10 nm und 5 µm, vorzugsweise zwischen 50 nm und 2 µm. Bezüglich des Filmbildungsverfahrens bei der Herstellung solcher Dünnfilmelektroden wird ein physikalisches oder chemisches Verfahren verwendet. Die physikalischen Verfahren sind als physikalisches Abscheidung (PVD) bekannt und enthalten Vakuumabscheidungsverfahren, ein Molekularstrahl-Epitaxieverfahren, ein Sputterverfahren, ein Ionisationsdampfabscheidungsverfahren und ein Laserabrasionsverfahren. Die chemischen Verfahren sind als chemische Dampfabscheidung (CVD) bekannt und enthalten eine thermische CVD, eine Plasma-CVD und eine MOCVD (metallorganische chemische Dampfabscheidung) usw. Von diesen Verfahren ist das Sputterverfahren besonders effektiv.

Der Begriff "flexibel" bedeutet im Zusammenhang mit der vorliegenden Erfindung, dass eine Schicht oder ein Substrat biegsam und insbesondere in bestimmten Grenzen deformierbar ist, ohne zu brechen oder jedenfalls ohne die gewünschten elektrischen und mechanischen Eigenschaften zu verlieren.

Unter der Bezeichnung "elektrisch leitfähig" wird nachfolgend verstanden, dass ein Material in der Lage ist, elektrischen Strom zu leiten und sich zur Ausbildung von Elektroden eignet. Neben der Leitfähigkeit, die beispielsweise Metalle an den Tag legen, soll damit im Sinne der vorliegenden Erfindung auch die Leitfähigkeit eines halbleitenden Materials umfasst sein.

Zum besseren Verständnis der vorliegenden Erfindung wird diese anhand der in den nachfolgenden Figuren dargestellten Ausführungsbeispiele näher erläutert. Dabei werden gleiche Teile mit gleichen Bezugszeichen und gleichen Bauteilbezeichnungen versehen. Weiterhin können auch einige Merkmale oder Merkmalskombinationen aus den gezeigten und beschriebenen unterschiedlichen Ausführungsformen für sich eigenständige, erfinderische oder erfindungsgemäße Lösungen darstellen. Es zeigen:
- **Fig. 1**: eine schematische Draufsicht auf eine Elektrodenanordnung gemäß einem ersten Beispiel der vorliegenden Erfindung;
- **Fig. 2**: eine schematische Draufsicht auf eine Elektrodenanordnung gemäß einem weiteren Beispiel der vorliegenden Erfindung mit nur teilweiser Darstellung der Fraktalisierung zur Erläuterung des Aufbaus der Anordnung;
- **Fig. 3**: eine schematische Draufsicht auf eine Elektrodenanordnung gemäß einem weiteren Beispiel der vorliegenden Erfindung;
- **Fig. 4**: eine schematische Draufsicht auf eine Elektrodenanordnung gemäß einem weiteren Beispiel der vorliegenden Erfindung;
- **Fig. 5**: eine schematische Draufsicht auf eine Elektrodenanordnung gemäß einem weiteren Beispiel der vorliegenden Erfindung;
- **Fig. 6**: eine erste Schnittdarstellung der Anordnung aus Fig. 5;
- **Fig. 7**: eine zweite Schnittdarstellung der Anordnung aus Fig. 5;
- **Fig. 8**: eine dritte Schnittdarstellung der Anordnung aus Fig. 5;
- **Fig. 9**: eine schematische Draufsicht auf eine Elektrodenanordnung gemäß einem weiteren Beispiel der vorliegenden Erfindung;
- **Fig. 10**: eine schematische Draufsicht auf eine Elektrodenanordnung gemäß einem weiteren Beispiel der vorliegenden Erfindung;
- **Fig. 11**: eine schematische Draufsicht auf eine Elektrodenanordnung gemäß einem weiteren Beispiel der vorliegenden Erfindung;
- **Fig. 12**: eine schematische Draufsicht auf eine Elektrodenanordnung gemäß einem weiteren Beispiel der vorliegenden Erfindung;
- **Fig. 13**: eine Schnittdarstellung eines Details der Anordnung aus Fig. 12;
- **Fig. 14**: eine schematische Draufsicht auf eine Elektrodenanordnung gemäß einem weiteren Beispiel der vorliegenden Erfindung;
- **Fig. 15**: eine schematische Draufsicht auf eine Elektrodenanordnung gemäß einem weiteren Beispiel der vorliegenden Erfindung;
- **Fig. 16**: eine schematische Draufsicht auf eine Elektrodenanordnung gemäß einem weiteren Beispiel der vorliegenden Erfindung;
- **Fig. 17**: eine schematische Draufsicht auf eine Elektrodenanordnung gemäß einem weiteren Beispiel der vorliegenden Erfindung;
- **Fig. 18**: eine schematische Draufsicht auf eine Elektrodenanordnung gemäß einem weiteren Beispiel der vorliegenden Erfindung;
- **Fig. 19**: eine schematische Draufsicht auf eine Elektrodenanordnung gemäß einem weiteren Beispiel der vorliegenden Erfindung;
- **Fig. 20**: eine schematische Draufsicht auf eine Elektrodenanordnung gemäß einem weiteren Beispiel der vorliegenden Erfindung;
- **Fig. 21**: eine schematische Draufsicht auf eine Elektrodenanordnung gemäß einem weiteren Beispiel der vorliegenden Erfindung;
- **Fig. 22**: eine schematische Draufsicht auf eine Elektrodenanordnung gemäß einem weiteren Beispiel der vorliegenden Erfindung;
- **Fig. 23**: eine schematische Draufsicht auf eine Elektrodenanordnung gemäß einem weiteren Beispiel der vorliegenden Erfindung;
- **Fig. 24**: eine schematische Draufsicht auf eine Elektrodenanordnung gemäß einem weiteren Beispiel der vorliegenden Erfindung;
- **Fig. 25**: eine schematische Schnittdarstellung der Anordnung aus Fig. 24;
- **Fig. 26**: eine schematische Draufsicht auf eine Elektrodenanordnung gemäß einem weiteren Beispiel der vorliegenden Erfindung;
- **Fig. 27**: eine schematische Schnittdarstellung der Anordnung aus Fig. 26;
- **Fig. 28**: eine schematische Draufsicht auf eine Elektrodenanordnung gemäß einem weiteren Beispiel der vorliegenden Erfindung;
- **Fig. 29**: eine schematische Schnittdarstellung der Anordnung aus Fig. 28;
- **Fig. 30**: eine schematische Darstellung der Auslenkung einer Elektrodenanordnung entlang einer Geraden über die Elektrodenfläche bei Stimulation mit einem Rechtecksignal.

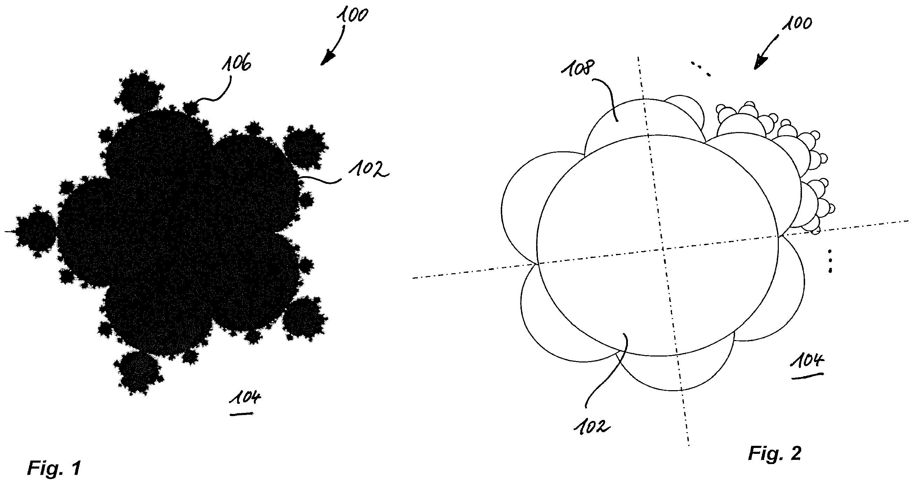

Die vorliegende Erfindung wird nachfolgend mit Bezug auf die Figuren, und dabei insbesondere zunächst mit Bezug auf die schematischen Schnittdarstellungen der Figur 1, näher erläutert. Es wird angemerkt, dass in sämtlichen Figuren die Größenverhältnisse und insbesondere die Schichtdickenverhältnisse nicht unbedingt maßstabsgetreu wiedergegeben sind. Auch der Grad der Fraktalisierung ist zur Beibehaltung der Übersichtlichkeit meist nicht in der tatsächlichen Tiefe und Strukturfeinheit wiedergegeben.

Figur 1 zeigt in einer Draufsicht eine beispielhafte Ausführungsform einer Elektrodenanordnung 100 gemäß einer ersten vorteilhaften Ausführungsform der vorliegenden Erfindung. Die Elektrodenanordnung 100 umfasst als elektrisch leitfähige Schicht eine Dünnfilmmetallisierung 102, die auf einer Trägerstruktur 104 abgeschieden ist. Die Dünnfilmmetallisierung 102 ist beispielsweise durch gesputtertes Platin gebildet und die Trägerstruktur 104 weist ein flexibles Polymer, beispielsweise Polyimid, auf. Für einen Fachmann ist selbstverständlich klar, dass auch andere elektrisch leitfähige Materialien als elektrisch leitfähige Schicht in Frage kommen und die Trägerstruktur 104 aus jedem beliebigen elektrisch isolierenden Material hergestellt werden kann.

Die Dünnfilmmetallisierung 102 ist so strukturiert, dass die mechanische Schwingung in Antwort auf ein angelegtes Feld reduziert wird, indem der Rand 106 durch Wiederholung immer kleiner werdender selbstähnlicher Elemente in Form einer fraktaler Mandelbrotstruktur fraktalisiert ist. Durch diese Diskontinuität einer glatten stetigen Umrissform der Elektrode kann erreicht werden, dass bei Anlegen eines elektrischen Feldes, beispielsweise eines sinusförmigen Wechselfeldes oder einer Spannungspulsfolge, die Dünnfilmmetallisierung 102 nicht in resonante Schwingungen versetzt wird und somit auch nicht so stark ausgelenkt wird, dass eine Delamination auftritt. Der Grad der fraktalisiert um, d.h. die Anzahl der selbstähnlichen Elemente, die aneinandergefügt werden, hängt selbstverständlich von den erreichbaren Strukturgenauigkeiten ab und endet in der Regel bei der vierten oder fünften Stufe.

Der in Figur 1 gezeigte fraktalisierte Elektrodenumriss wird vorzugsweise so gewählt, dass der größte auftretende Durchmesser kompatibel ist mit üblichen Elektrodenflächen, so dass beim Freilegen der Elektrode durch Entfernen einer Deckschicht keine Technologieänderung erforderlich ist.

Figur 2 illustriert schematisch den Aufbau einer Elektrodenanordnung 100 ähnlich der aus Figur 1, wobei in dieser Darstellung lediglich ein Teil des Elektrodenrands 106 so gezeigt ist, dass er sichelförmige Fortsätze 108 bis in die vierte Verkleinerungsstufe beinhaltet. Selbstverständlich ist die Elektrodenanordnung 100 tatsächlich umlaufend mit den sichelförmigen Fortsätzen ausgestatten. Die Verkleinerungsstufe kann dabei soweit gehen, wie dies für das gewünschte Ziel erforderlich und von den herstellbaren Strukturabmessungen machbar ist. In Figur 2 sind an die kreisförmige Struktur sechs sichelförmige Fortsätze 108 angebracht, die jeweils wieder jeder drei Fortsätze tragen. Demgegenüber besitzt die Elektrodenanordnung der Fig. 1 eine fünfzählige Struktur. Es ist aber für einen Fachmann klar, dass auch andere Anzahlen von Fortsätzen gewählt werden können. Durch diese diskontinuierliche Ausgestaltung des Elektrodenumrisses wird die Resonanzfähigkeit der Dünnfilmmetallisierung 102 bei Anregung mit einem Anregungssignal wie beispielsweise einer Spannungspulsfolge oder einer Strompulsfolge mit Frequenzen zwischen 1 kHz und 10 kHz reduziert, so dass die bei bekannten Elektrodenanordnungen auftretenden Ablösungseffekte verhindert werden können.

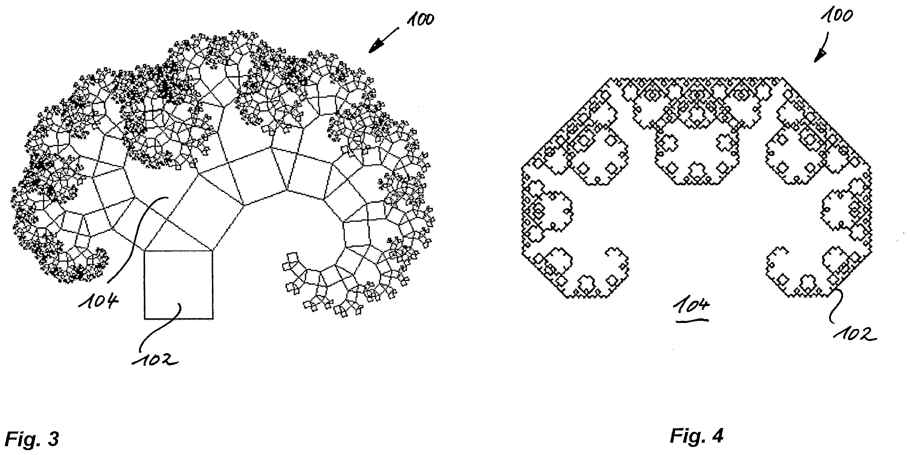

Wie in Figur 3 gezeigt, eignet sich für die Erzeugung einer Fraktalisierung des Elektrodenrandes auch ein Fraktal basierend auf dem Pythagorasbaum, das in Figur 3 dargestellt ist. Ausgangspunkt ist das untere rechtwinklige Dreieck. An dieses Dreieck werden die Quadrate über der Hypotenuse und den Katheten gezeichnet. An die Kathetenquadrate wird jeweils ein weiteres Dreieck konstruiert, welches dem ersten Dreieck ähnlich ist. An deren Katheten werden wieder die Quadrate ergänzt - Stufe 2 ist erreicht. Nach diesem Verfahren wird Schritt für Schritt weiter verfahren. Anstelle eines Quadrats können auch andere Formen, z. B. regelmäßige Sechsecke angefügt werden.

Eine weitere Möglichkeit, den Elektrodenrand zu fraktalisieren, besteht in der Verwendung einer Levy-C-Kurve, die in Figur 4 gezeigt ist.

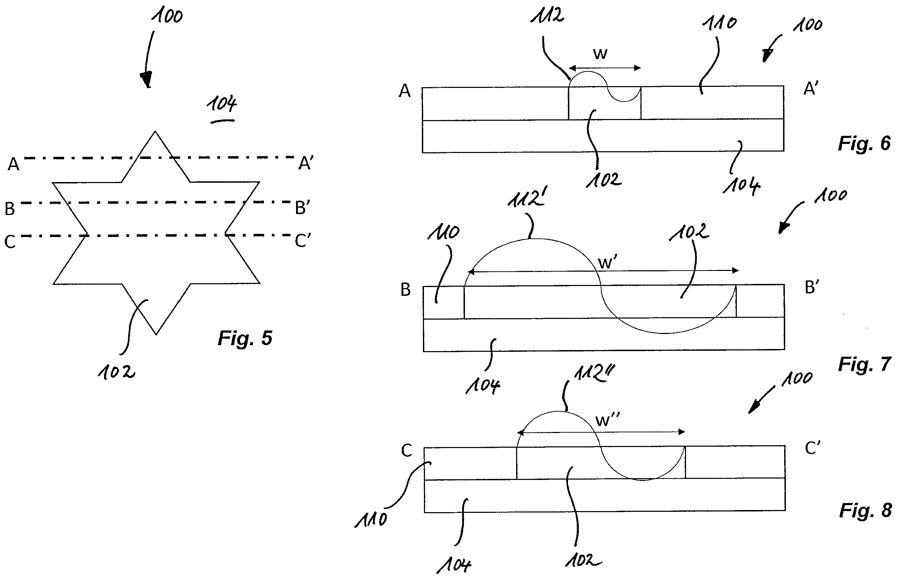

Weiterhin kann auch der lokale Durchmesser variiert werden. Vorteilhafte Umrissausgestaltungen einer Elektrodenanordnung 100 basierend auf dem sogenannten Koch-Stern oder der Koch-Schneeflocke sind in den Figuren 5 bis 11 illustriert.

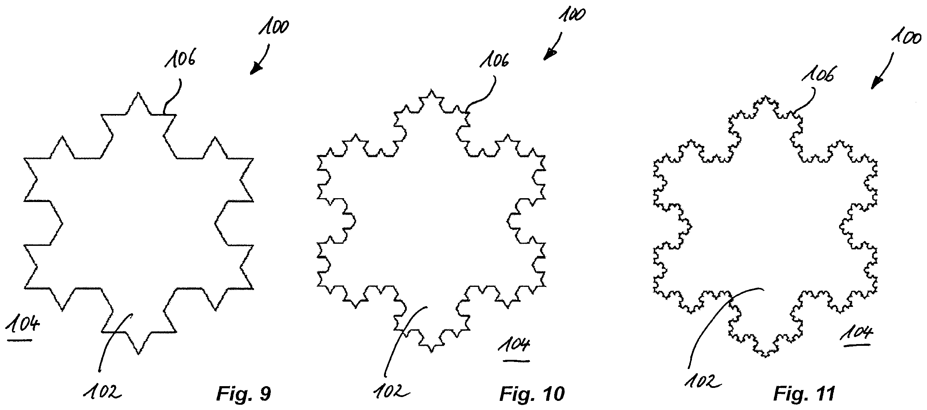

Wie zunächst in Figur 5 gezeigt, hat die elektrisch leitfähige Schicht 102 einen sternförmigen Umriss. Die sogenannte Kochsche Kurve (auch Schneeflockenkurve genannt) ist in der einfachsten Form ein gleichseitiges Dreieck. Jede Seite wird gedrittelt und über dem Mittelstück ein gleichseitiges Dreieck nach außen angesetzt. Analog kann man in den weiteren Schritten mit jedem geraden Stück vorgehen, wie dies in den Figuren 9 bis 11 gezeigt ist. Figur 5 zeigt die erste Fraktalisierungsstufe, bei der ein sechszackiger Stern vorliegt.

Die Figuren 6, 7 und 8 zeigen Schnitte entlang der Schnittlinien A-A`, B-B' beziehungsweise C-C`. Aus diesen Schnittdarstellungen ist erkennbar, dass die elektrisch leitfähige Struktur 102 in einer Deckschicht 110 eingebettet ist. Die Deckschicht 110 ist vorzugsweise aus demselben Material wie die auch als Substrat bezeichnete Trägerstruktur 104. Erfindungsgemäß unterscheiden sich die Wellenlängen der möglichen Resonanzschwingungen aufgrund der unterschiedlichen lokalen Durchmesserw, w` und w" voneinander. Die Schwingungskurven 112, 112' und 112" sind schematisch für den jeweiligen Durchmesser w, w` und w" eingezeichnet. Damit unterscheidet sich lokal das Verhältnis des Elektrodendurchmessers zur Gesamtfläche wie auch zur Querschnittsfläche und es wird verhindert, dass eine globale Resonanz der Dünnfilmmetallisierung 102 auftritt.

Die Figuren 9 bis 11 illustrieren weitere Stufen der Kochschen Schneeflocke mit immer feinerer Auffaserung des Elektrodenrandes durch Anwendung der obigen mathematischen Anweisung. Es ist für einen Fachmann klar, dass beliebige Fraktalisierungsstufen verwendet werden können, je nach erreichbarer Strukturfeinheit und dem sich einstellenden Effekt der Resonanzreduktion. Allgemein können auch andere (hier nicht dargestellte) Umrissfraktalisierungen, wie z. B. Kepler-Fraktale verwendet werden.

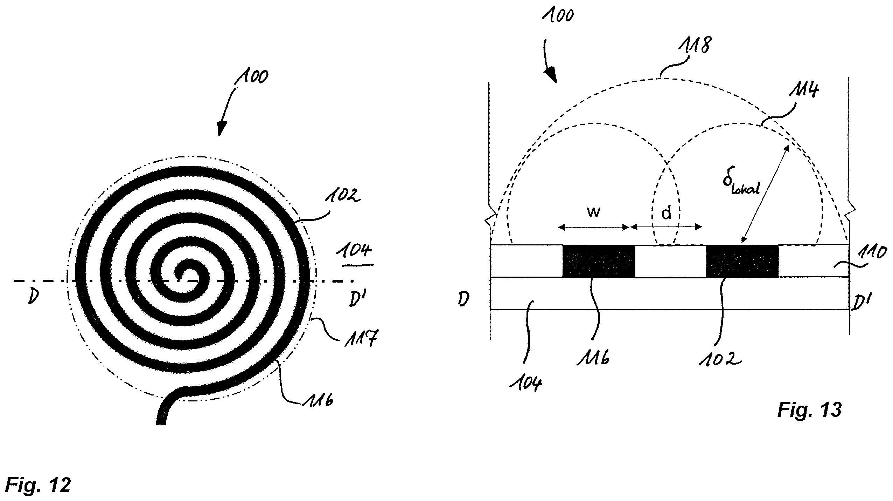

Gemäß einer weiteren vorteilhaften Ausführungsform, die nachfolgend mit Bezug auf die Figuren 12 bis 23 erläutert werden soll, kann auch vorgesehen sein, dass die Elektrodenfläche streifenförmig ausgestaltet und durch ein elektrisch isolierendes Material unterbrochen ist. Damit wird die geometrische Fläche der Dünnschichtmetallisierung so modifiziert, dass die mechanische Resonanz bei Einwirken einer Anregungsspannung minimal ist. Bei dieser Ausführungsform bleibt das Verhältnis zwischen dem lokalen Elektrodendurchmesser w und der Gesamtfläche (wie auch der Querschnittsfläche) über die gesamte Elektrodenanordnung 100 konstant. Beispielsweise kann die Elektrodenfläche 102 durch einen spiralförmig ausgestalteten leitfähigen Streifen 116 gebildet sein, wie die in Figur 12 illustriert ist. Wie durch die strichpunktierte kreisförmige Linie 117 angedeutet, ist die Leiterbahnspirale einer Öffnung in der isolierenden Deckschicht einbeschrieben. Damit kann die aktive Elektrodenfläche beispielsweise den üblichen Durchmessern entsprechen und es müssen keine Anpassung an den Vorrichtungen vorgenommen werden, die mit der Dünnschichtelektrode arbeiten. Ein Ende der Leiterbahnspirale ist als elektrischer Anschluss zum Einspeisen des elektrischen Signals ausgebildet, während das andere Ende offenbleibt.

Figur 13 illustriert als Detail des Querschnitts durch die Draufsicht der Figur 12 die auftretenden elektrischen Felder. Jeder streifenförmige Teil 116 der elektrisch leitfähigen Schicht 102 ist von einem elektrischen Feld 114 umgeben, das zu einem lokalen lonenstrom δₗₒₖₐₗ führt. Die Leiterbahnbreite w und die Abstände d der leitfähigen Teile voneinander werden zweckmäßigerweise so gewählt, dass sich die lokalen lonenströme und die lokalen elektrischen Felder zu einem homogenen globalen elektrischen Feld addieren.

Mit anderen Worten entsteht ein lokaler Mikroelektrodeneffekt mit sich überlagernden elektrischen Feldern 114 und somit überlagernden lonenströmen δₗₒₖₐₗ ergibt, wobei sich diese lokalen elektrischen Feldkomponenten 114 wieder zu einem globalen homogenen elektrischen Feld 118 in Bezug auf den Elektrodenausschnitt 117 im Substrat (gemeint ist hier der äußere Durchmesser/Öffnung/Grundgeometrie der Elektrode) überlagern sollen. Mit der Fraktalisierung der Elektrodenfläche wird die lokale Änderung der mechanischen Schwingungseigenschaften bei gleichbleibendem globalem Wirkungsradius der Elektrode zur Anpassung der lokalen mechanischen Schwingung und globalen Resonanzminimierung erzielt.

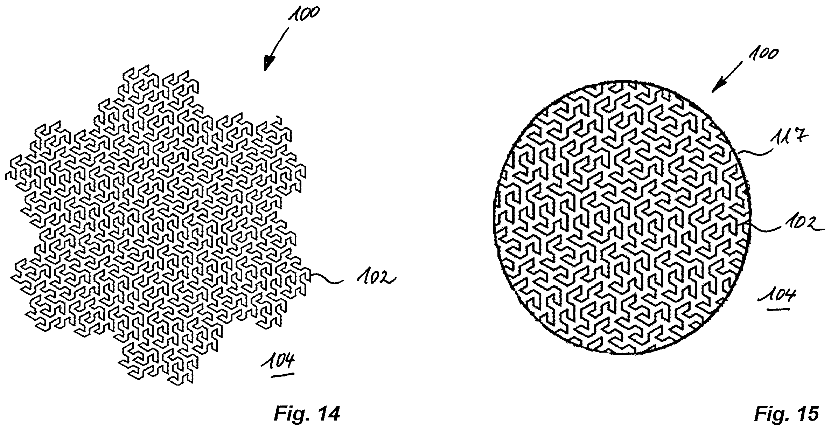

Die in Figur 12 gezeigte Spiralstruktur ist nur eine von vielen Möglichkeiten, die Dünnfilmmetallisierung 102 in definierter Weise zu unterbrechen. Wie in Figur 14 gezeigt, kann die Leiterbahn 116 die Gestalt einer sogenannten Gosperkurve haben. Die Gosperkurve (benannt nach Bill Gosper) ist ein fraktales Objekt, das durch Ersetzung von Streckenstücken erzeugt wird. Die in Figur 14 gezeigte Kurve ist beispielsweise eine Gosperkurve der Stufe 4. Andere Stufen können selbstverständlich auch verwendet werden. Alternativ kann auch die Gosperkurve die Lage der isolierenden Bereiche 104 definieren und die in Figur 14 weißen Bereiche sind elektrisch leitfähig.

Wie in Figur 15 gezeigt, kann die fraktalisierte Elektrodenfläche auch durch eine nicht von der Fraktalkurve bestimmte Randform haben. Dies kann beispielsweise eine kreisförmige Begrenzung sein. Andere Ausgestaltungen der Außenbegrenzung 117, wie z. B. ein polygonaler Umriss, sind selbstverständlich auch möglich.

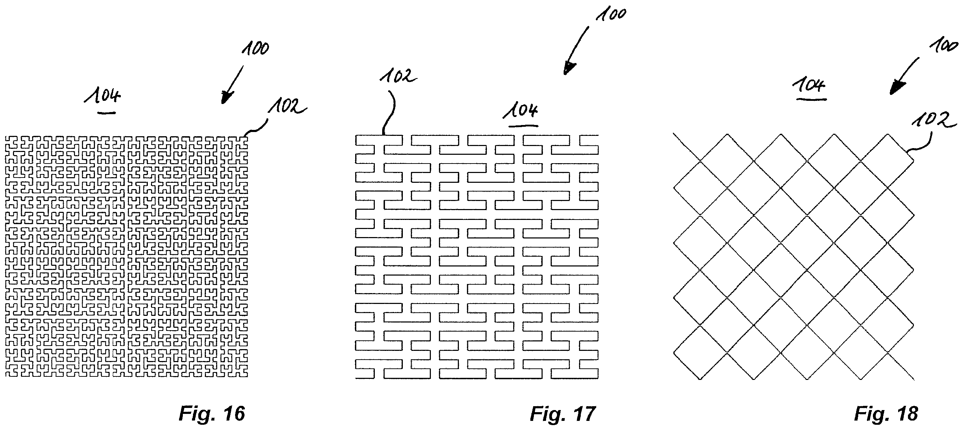

Die Figuren 16 bis 18 illustrieren weitere Beispiele für flächig fraktalisierte Dünnfilmmetallisierungen 102. So zeigt Figur 16 eine Hilbert-Fraktalisierung und Figur 17 eine Peano-Fraktalisierung. Die Peano-Kurve ist eine selbstähnliche ebenenfüllende Kurve mit der fraktalen Dimension 2. Figur 18 zeigt ein weiteres Beispiel einer Peano-Kurve, die als 1:1/3 Fraktalisierung bezeichnet wird. Obwohl in den Figuren 16 bis 18 schematisch eine rechteckige Außenkontur der Elektrode gezeigt ist, kann die Dünnfilmmetallisierung 102 jede beliebige Außenkontur, z. B. eine kreisförmige Kontur, wie in Fig. 15 gezeigt, haben.

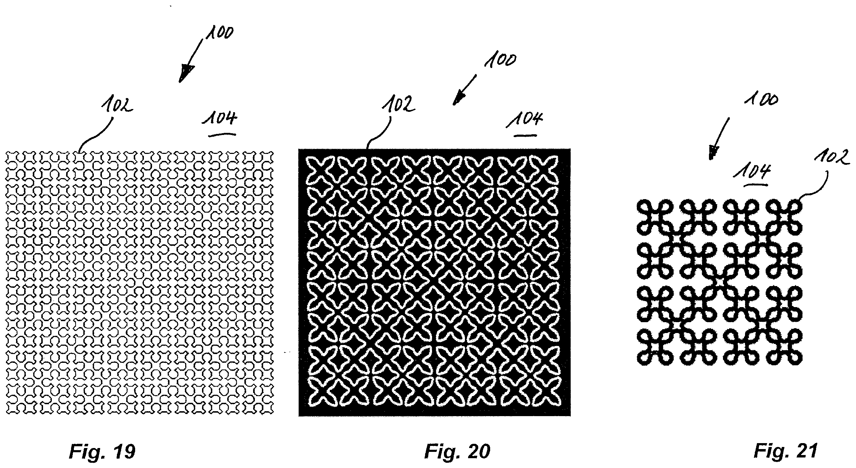

Weiterhin sind auch sogenannte Sierpinski-Fraktale geeignet, unterbrochene Dünnfilmmetallisierungen zu erzeugen, die ein optimiertes Resonanzverhalten bei Anregung mit Strom- oder Spannungspulsen aufweisen. Die Sierpinski-Kurven, die in den Figuren 19 bis 21 gezeigt sind, können dabei so wie abgebildet ausgeführt sein, so dass die leitfähigen Bereiche durch die dunkel dargestellten Bereiche gebildet sind. Alternativ kann das Muster auch invertiert sein, so dass die hell dargestellten Bereiche elektrisch leitfähig sind. Sierpinski-Fraktale entstehen, wie dies bekannt ist, nach verschiedenen Konstruktionsvorschriften durch wiederholte Unterteilung einer gegebenen Ausgangsform. Obwohl in den Figuren 19 bis 21 schematisch eine rechteckige Außenkontur gezeigt ist, kann die Dünnfilmmetallisierung 102 jede beliebige Außenkontur, z. B. eine kreisförmige Kontur, wie in Fig. 15 gezeigt, haben.

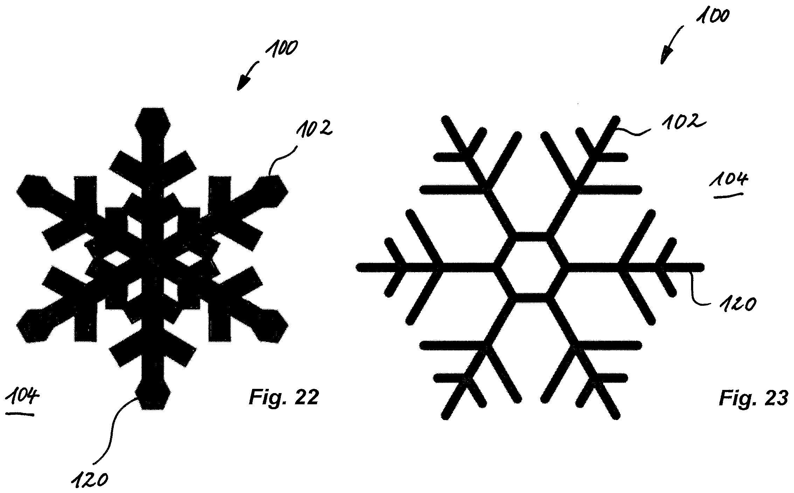

Zwei weitere mögliche Realisierungsformen solcher unterbrochener Dünnfilmmetallisierungen 102 sind in den Figuren 22 und 23 gezeigt. In beiden Fällen hat die elektrisch leitfähige Schicht der Elektrodenanordnung 100 eine schneeflockenförmige Geometrie. In Figur 22 ist dabei eine lokal variierende Breite der leitfähigen Bahnen 120 gezeigt, während in Figur 23 eine im wesentlichen konstante Breite der Bahnen 120 gezeigt ist.

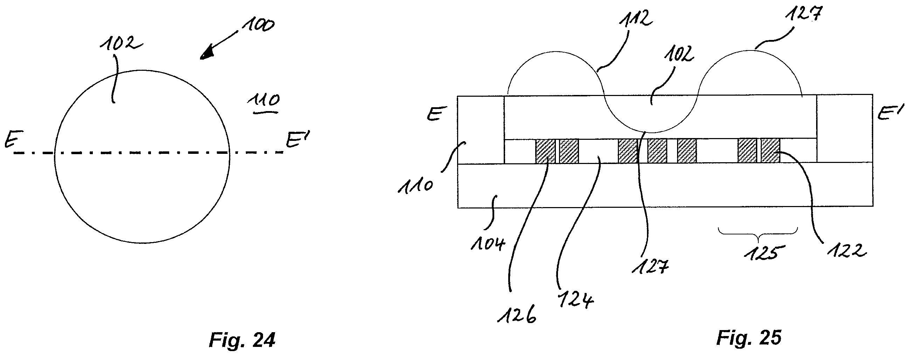

Eine weitere vorteilhafte Ausführungsform der vorliegenden Erfindung wird nachfolgend mit Bezug auf die Figuren 24 und 25 näher erläutert.

Fig. 24 zeigt eine schematische Draufsicht auf eine Elektrodenanordnung 100 mit einer kreisförmigen geschlossenen Dünnfilmmetallisierung, die von ihren Abmessungen den in den obigen Artikel erwähnten bekannten implantierbaren Elektroden entspricht. Die elektrisch leitfähige Schicht 102 ist auf einer Trägerstruktur 104 (auch als "Substrat" bezeichnet) in einer Ausnehmung einer Deckschicht 110 eingebettet, wie dies aus der Schnittdarstellung der Fig. 25 ersichtlich ist. Eine Haftvermittlerschicht 122 ist zwischen der elektrisch leitfähigen Schicht 102 und der Trägerstruktur 104 angeordnet. Erfindungsgemäß ist diese Haftvermittlerschicht 122 nicht kontinuierlich durchgehend ausgebildet, sondern strukturiert. Die Haftvermittlerschicht weist insbesondere Unterbrechungen 124 auf, die mit sogenannten Bumps 126 abwechseln. Diese Unterbrechungen 124 werden von der Dünnfilmmetallisierung 102 aufgefüllt.

Durch eine solche Fraktalisierung der Elektrodenhaftvermittlerschicht 122 zwischen Oberflächenmetallisierung 102 und Substrat 104 erfolgt eine lokale Versteifung z. B. in dem Bereich 125 und es werden lokale Ankerpunkte (genannt Bumps 126) zwischen Metallisierung 102 und Substrat 104 als lokale Adhäsionspromoter gesetzt. Erfindungsgemäß werden die Bumps 126 an den lokalen Stellen der mechanischen Schwingungsextrema 127 der Dünnfilmmetallisierung 102 angeordnet. Dadurch kann einerseits durch lokale Verstärkung über die Adhäsion an Stellen der ansonsten auftretenden Schwingungsmaxima 127 die Auslenkung reduziert werden. Andererseits wird an den Orten der Unterbrechungen 124 durch lokale Nicht-adhäsion (durch Nichtsetzen von Bumps 126) an den Stellen, an denen bei der nichtmodifizierten Elektrodenanordnung Schwingungsminima, d. h. Nulldurchgänge der eingezeichneten Schwingungskurve 112, auftreten, eine lokale Schwächung (d. h. keine Adhäsion) der Dünnfilmmetallisierung 102 erreicht. Global über die gesamte Elektrodenfläche verteilt wird dadurch die mechanische Resonanzantwort reduziert.

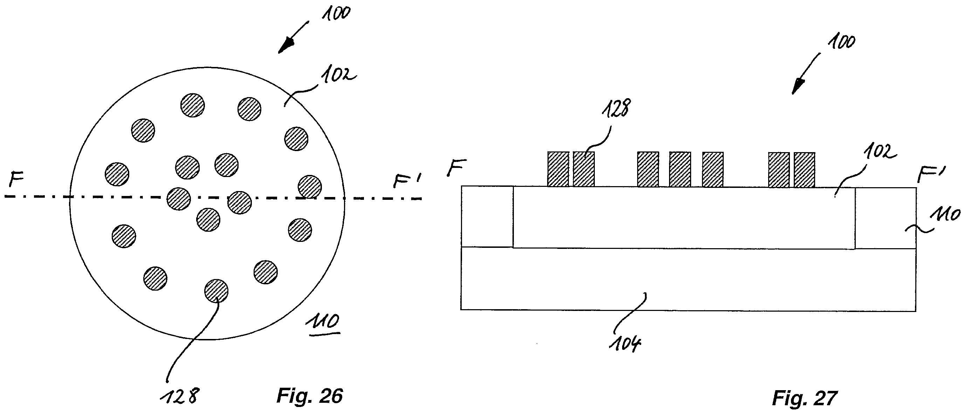

Eine weitere Ausführungsform einer Elektrodenanordnung 100 gemäß der vorliegenden Erfindung wird im Folgenden mit Bezug auf die Figuren 26 und 27 erläutert. Gemäß dieser Ausführungsform ist eine kreisförmige Dünnfilmmetallisierung 102 auf der Trägerstruktur 104 aufgebracht und in einer Deckschicht 110 eingebettet. Auf der Oberfläche der Dünnfilmmetallisierung 102 sind Erhebungen 128 angeordnet, die beispielsweise durch Hügelstrukturen gebildet sind. Diese Erhebungen 128 bilden eine fraktalisierte dreidimensionale Strukturierung der Dünnfilmoberfläche aus. Die Erhebungen 128 führen zu lokalen Verstärkungen der Dünnfilmmetallisierung 102 an den Orten, an denen bei der nicht modifizierten dünnen Filmmetallisierung Schwingungsextrema auftreten. An dieser Stelle soll nochmals betont werden, dass die Darstellung der Erhebungen 128 stark schematisch ist, weil die Strukturen in Realität sehr viel feiner ausgebildet sind. Weiterhin ist die dargestellte Platzierung der Erhebungen 128 auch nur beispielhaft skizziert.

Die Erhebungen 128 können aus den verschiedensten Materialien hergestellt sein. Zum einen können sie Teil der Metallisierung selbst sein. Zum anderen können auch Polymerstrukturen oder andere geeignete Materialien verwendet werden.

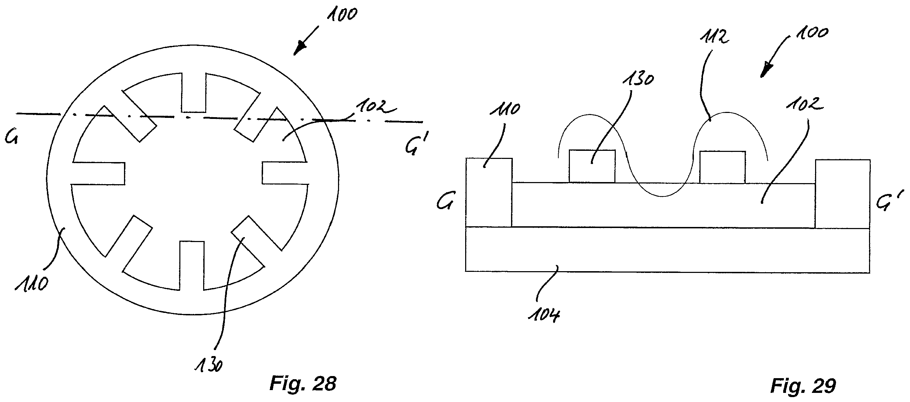

Eine Dämpfung der mechanischen Schwingungen (angedeutet durch die Schwingungskurve 112) kann auch durch strukturierte Elemente der Deckschicht 110 erzielt werden. Ein Beispiel für diese Art der Kontinuitätsunterbrechung ist in den Figuren 28 und 29 dargestellt. Dabei ist die Dünnfilmmetallisierung 102 auf einer Trägerstruktur 104 abgeschieden und in einer Deckschicht 110 eingebettet. Gemäß der hier gezeigten Ausführungsform weist die Deckschicht fingerförmige Stege 130 auf, die sich radial in Richtung auf das Innere der elektrisch leitfähigen Schicht erstrecken. In dem gezeigten Beispiel sind acht gleichmäßig um den Umfang und symmetrisch verteilte Stege 130 vorgesehen. Es ist aber für einen Fachmann selbstverständlich klar, dass beliebige andere Anzahlen von Stegen 130 und auch eine unsymmetrische Verteilung ebenfalls vorgesehen sein kann.

Die Stege 130 können dabei auf der Dünnfilmelektrode 102 nur aufliegen oder fest haften. Damit erfolgt eine lokale mechanische Schwingungsdämpfung durch die Deckschicht 110 von der Oberseite aus.

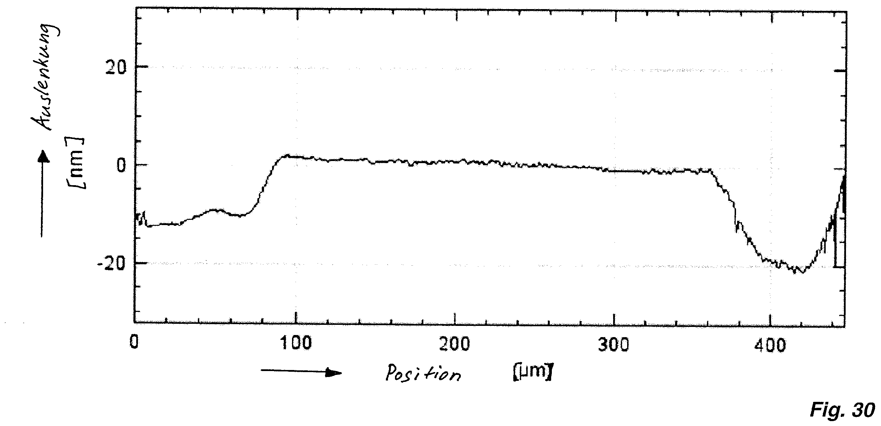

Beim Entwurf derartiger Elektrodenstrukturen wird zunächst die Resonanzantwort einer Dünnfilmmetallisierung 102 mit den gewünschten Außenabmessungen für die relevanten Anregungssignale ermittelt. Fig. 30 zeigt beispielhaft die Auslenkung einer Platindünnfilmelektrode (in Nanometern) über die gesamte Elektrodenoberfläche entlang eines zentralen Durchmessers (in Mikrometern), wie sie zum Beispiel als mechanische Antwort auf eine elektrische Stimulation mit einem Anregungssignal (z. B. einem Rechtecksignal) im Bereich von 1 Hz bis 100 kHz auftreten.

Dann werden z. B. die entsprechenden Orte zum Setzen von Haftvermittlerbumps 126 und Unterbrechungen 124 oder die Art einer der anderen oben beschriebenen Modifikationen bestimmt. Bei einer anschließenden Überprüfung kann festgestellt werden, ob der gewünschte Effekt erreicht wurde oder noch weitere Modifikationen der Dünnfilmmetallisierung vorgenommen werden sollen.

Obwohl dies in den Figuren nicht gezeigt ist, kann die lokale Fraktalisierung eine über die Elektrodenfläche verteilte Variation einer Defektdichte in dem elektrisch leitfähigen Material der Elektrode umfassen. Durch das Anpassen der Defektdichte wird der intrinsische Schichtstress im Dünnfilmmikrogefüge angepasst, so dass das Resonanzverhalten optimiert wird. Die Defektdichte kann z. B. durch Anpassen der Fertigungsparameter von gesputterten und aufgedampften Dünnfilmschichten gesteuert werden, so dass ein Resonanzverhalten minimiert wird.

Dabei kann das Ermitteln der Resonanzantwort sowohl durch tatsächliche Messung, z. B. mittels digitaler Holographie während der elektrischen Stimulation, wie auch durch geeignete Simulationsverfahren erfolgen. Vorzugsweise wird die mechanische Auslenkung der Dünnfilmelektroden als Antwort auf ein angelegtes elektrisches Wechselfeld mittels digitaler Holographie in situ zeitgleich während der Stimulation aufgenommen. Die mechanische Deformation, die Schwingungsamplitude und/oder die räumlich aufgelöste Schwingungsphase sowie ein eventueller mechanischer Ausfall der Elektrode nach erfolgter Stimulation können mit Hilfe eines normalen optischen Licht-Mikroskops in Aufsicht aufgenommen werden.

Mit diesem Untersuchungsansatz können Dünnfilmelektroden auf ihr Resonanzverhalten infolge der elektrischen Stimulation für verschiedenste Anwendungen im Bereich der Neuroprothetik untersucht und angepasst werden und mechanisch langzeitstabil entworfen werden.

Zusammenfassend stellt die vorliegende Erfindung eine Möglichkeit bereit, die Langzeitstabilität von implantierbaren Elektrodenanordnungen zu erhöhen, indem eine Schwingungskompensation durch das Elektrodendesign erfolgt. Insbesondere erfolgt eine Fraktalisierung der lokalen Dünnfilmgeometrie und der lokalen Dünnfilmfläche, des Dünnfilmrandbereichs, der Dünnfilmtopografie (durch Hügelstrukturen, Bumps oder "Eierkarton"-Strukturen) für eine globale homogene elektrische Feldverteilung und mechanische Schwingung (Resonanz der Elektrode), oder der Haftvermittlertopografie durch lokale mechanische Schwächung und Stärkung des Dünnfilms und Substratoberseitentopografie zur lokalen Dämpfung des Dünnfilms an den Schwingungsextrema.

Weiterhin sollte hervorgehoben werden, dass mehrere oder alle der oben beschriebenen Fraktalisierungsmechanismen auch beliebig miteinander kombiniert werden können, um die gewünschte Resonanzarmut der Elektrode zu erreichen.

### Bezugszeichenliste:

| **Bezugsziffer** | **Beschreibung** |
|---|---|
| 100 | Elektrodenanordnung |
| 102 | Dünnfilmmetallisierung, elektrisch leitfähige Schicht; Elektrode |
| 104 | Trägerstruktur |
| 106 | Elektrodenrand |
| 108 | Einzelelement eines fraktalen Umrisses |
| 110 | Deckschicht |
| 112 | Schwingungskurve |
| 114 | Lokales elektrisches Feld |
| 116 | Streifenförmiger Teil des elektrisch leitfähigen Schicht |
| 117 | Umriss der Öffnung im Isolator, Standardelektrodenabmessung |
| 118 | Globales elektrisches Feld |
| 120 | Leitfähige Bahn |
| 122 | Haftvermittlerschicht |
| 124 | Unterbrechungen der Haftvermittlerschicht |
| 125 | Bereich lokaler Versteifung |
| 126 | Haftvermittlerbumps |
| 127 | Schwingungsextrema |
| 128 | Erhebungen auf der Dünnfilmmetallisierung |
| 130 | Steg |

## Patentansprüche

1. Verfahren zum Entwerfen einer Elektrodenanordnung, wobei das Verfahren die folgenden Schritte umfasst:
Bereitstellen einer Startkonfiguration einer flexiblen implantierbaren Elektrodenanordnung (100) mit einer elektrisch isolierenden Trägerstruktur (104) und einer elektrisch leitfähigen Schicht (102), die eine elektrisch leitfähige Dünnfilmschicht aufweist, wobei die elektrisch leitfähige Dünnfilmschicht strukturiert ist, um wenigstens eine implantierbare Elektrode auszubilden;
Anregen der Elektrodenanordnung (100) mit einer alternierenden Anregungsspannung oder einem alternierenden Anregungsstrom;
Ermitteln einer mechanischen Auslenkung der Elektrode (102) in Antwort auf die Anregung;
Modifizieren der Elektrodenanordnung (100), so dass die mechanische Auslenkung der Elektrode (102) in Antwort auf die elektrische Anregung ein Minimum annimmt.

2. Implantierbare Elektrodenanordnung (100) umfassend:
eine elektrisch isolierende Trägerstruktur (104), und
eine elektrisch leitfähige Schicht, die eine elektrisch leitfähige Dünnfilmschicht aufweist, wobei die elektrisch leitfähige Dünnfilmschicht strukturiert ist, um wenigstens eine implantierbare Elektrode (102) auszubilden,
wobei die wenigstens eine Elektrode (102) eine lokale Fraktalisierung durch selbstähnliche Strukturierung aufweist,
**dadurch gekennzeichnet,**
**dass** die lokale Fraktalisierung unter Verwendung des Verfahrens nach Anspruch 1 in dem Schritt des Modifizierens der Elektrodenanordnung (100) gewählt ist.

3. Elektrodenanordnung nach Anspruch 2, wobei die lokale Fraktalisierung eine Strukturierung eines Umrisses der Elektrode (102) umfasst, so dass eine Elektrodenfläche durch eine selbstähnliche Mandelbrotgeometrie gebildet ist.

4. Elektrodenanordnung nach Anspruch 2 oder 3, wobei die lokale Fraktalisierung eine Strukturierung des Umrisses der Elektrode umfasst, so dass lokal unterschiedliche Verhältnisse zwischen einem Elektrodendurchmesser und der Elektrodenfläche eingestellt sind.

5. Elektrodenanordnung nach Anspruch 4, wobei der Elektrodenumriss eine sternförmige oder schneeflockenähnliche Geometrie hat.

6. Elektrodenanordnung nach einem der Ansprüche 2 bis 5, wobei die lokale Fraktalisierung eine Unterbrechung der nach außen wirksamen elektrisch leitfähigen Schicht umfasst.

7. Elektrodenanordnung nach Anspruch 6, wobei die Unterbrechung durch teilweises Abdecken der elektrisch leitfähigen Schicht (102) mit einer elektrisch isolierenden Schicht (110) ausgebildet ist.

8. Elektrodenanordnung nach Anspruch 6 oder 7, wobei die Unterbrechung durch Einbetten elektrisch leitfähiger Bahnen (120) in die elektrisch isolierende Trägerstruktur (104) ausgebildet ist.

9. Elektrodenanordnung nach einem der Ansprüche 2 bis 8, wobei zwischen der elektrisch isolierenden Trägerstruktur (104) und der elektrisch leitfähigen Schicht (102) eine Haftvermittlerschicht (122) angeordnet ist und die lokale Fraktalisierung eine Strukturierung der Haftvermittlerschicht (122) umfasst, so dass die Haftung über die Elektrodenfläche verteilt variiert.

10. Elektrodenanordnung nach einem der Ansprüche 2 bis 9, wobei die lokale Fraktalisierung eine über die Elektrodenfläche verteilte Variation der Dicke der Dünnfilmschicht (102) aufweist.

11. Elektrodenanordnung nach Anspruch 10, wobei die Elektrodenoberfläche mit einer Vielzahl von Erhebungen (128), die über die Elektrodenfläche verteilt sind, lokal verstärkt ist.

12. Elektrodenanordnung nach einem der Ansprüche 2 bis 11, wobei die lokale Fraktalisierung eine über die Elektrodenfläche verteilte Variation einer Defektdichte in dem elektrisch leitfähigen Material der Elektrode umfasst.

13. Elektrodenanordnung nach einem der Ansprüche 2 bis 12, wobei die elektrisch isolierende Trägerstruktur (104) ein Polymermaterial aufweist.

14. Elektrodenanordnung nach einem der Ansprüche 2 bis 13, wobei die Dünnfilmschicht (102) Platin, Iridium, und/oder Iridiumoxid aufweist und/oder eine Elektrodenbeschichtung aus Metallen, Metalloxiden, Metallnitriden oder Kohlenstoffschichten vorliegt.

## Claims

1. A method of designing an electrode arrangement, the method comprising the following steps:
providing a start configuration of a flexible implantable electrode arrangement (100) comprising an electrically insulating carrier structure (104) and an electrically conductive layer (102), which includes an electrically conductive thin film layer, the electrically conductive thin film layer being structured so as to form at least one implantable electrode;
exciting the electrode arrangement (100) with an alternating excitation voltage or an alternating excitation current;
determining a mechanical deflection of the electrode (102) in response to the excitation;
modifying the electrode arrangement (100) so that a mechanical resonance of the electrode (102) in response to the electric excitation will assume a minimum.

2. An implantable electrode arrangement (100) comprising:
an electrically insulating carrier structure (104), and
an electrically conductive layer including an electrically conductive thin film layer, the electrically conductive thin film layer being structured so as to form at least one implantable electrode (102),
wherein the at least one electrode (102) exhibits a local fractalization through self-similar structuring,
**characterized in that**
the local fractalization is chosen using the method according to claim 1 in the step of modifying the electrode arrangement (100).

3. The electrode arrangement according to claim 2, wherein the local fractalization includes a structuring of a contour of the electrode (102), so that an electrode area is defined by a self-similar Mandelbrot geometry.

4. The electrode arrangement according to claim 2 or 3, wherein the local fractalization includes a structuring of the contour of the electrode, so that locally different conditions between an electrode diameter and the electrode area are set.

5. The electrode arrangement according to claim 4, wherein the electrode contour has a star-shaped or a snowflake-like geometry.

6. The electrode arrangement according to one of the claims 2 to 5, wherein the local fractalization includes an interruption of the electrically conductive layer which is effective towards the outside.

7. The electrode arrangement according to claim 6, wherein the interruption is formed by partially covering the electrically conductive layer (102) with an electrically insulating layer (110).

8. The electrode arrangement according to claim 6 or 7, wherein the interruption is formed by embedding electrically conductive paths (120) into the electrically insulating carrier structure (104).

9. The electrode arrangement according to one of the claims 2 to 8, wherein an adhesion promoting layer (122) is arranged between the electrically insulating carrier structure (104) and the electrically conductive layer (102), and the local fractalization includes a structuring of the adhesion promoting layer (122), so that the adhesion varies over the electrode area in a distributed manner.

10. The electrode arrangement according to one of the claims 2 to 9, wherein the local fractalization exhibits, distributed over the electrode area, a variation of the thickness of the thin film layer (102).

11. The electrode arrangement according to claim 10, wherein the electrode surface is locally strengthened by a large number of elevations (128) distributed over the electrode area.

12. The electrode arrangement according to one of the claims 2 to 11, wherein the local fractalization includes, distributed over the electrode area, a variation of a defect density in the electrically conductive material of the electrode.

13. The electrode arrangement according to one of the claims 2 to 12, wherein the electrically insulating carrier structure (104) comprises a polymer material.

14. The electrode arrangement according to one of the claims 2 to 13, wherein the thin film layer (102) comprises platinum, iridium and/or iridium oxide and/or the electrode is coated with metals, metal oxides, metal nitrides or carbon layers.

## Revendications

1. Procédé de conception d'un ensemble d'électrodes, le procédé comprenant les étapes suivantes consistant à:
fournir une configuration de départ d'un ensemble d'électrodes implantables flexible (100) comprenant une structure de support électriquement isolante (104) et une couche électriquement conductrice (102) comprenant une couche de film mince électriquement conductrice, dans lequel la couche de film mince électriquement conductrice est structurée pour former au moins une électrode implantable ;
exciter l'ensemble d'électrodes (100) avec une tension d'excitation ou un courant d'excitation alterné(e) ;
déterminer une déviation mécanique de l'électrode (102) en réponse à l'excitation ;
modifier l'ensemble d'électrodes (100) de sorte que la déviation mécanique de l'électrode (102) en réponse à l'excitation électrique prenne une valeur minimale.

2. Ensemble d'électrodes implantables (100) comprenant
une structure de support électriquement isolante (104), et
une couche électroconductrice comprenant une couche de film mince électroconductrice, dans laquelle la couche de film mince électroconductrice est structurée pour former au moins une électrode implantable (102),
dans lequel ladite au moins une électrode (102) présente une fractalisation locale par structuration auto-similaire,
**caractérisé en ce que**
la fractalisation locale est choisie en utilisant le procédé selon la revendication 1 dans l'étape de modification de l'ensemble d'électrodes (100).

3. Ensemble d'électrodes selon la revendication 2, dans lequel la fractalisation locale comprend une structuration d'un contour de l'électrode (102) de sorte qu'une surface de l'électrode est formée selon une géométrie de Mandelbrot auto-similaire.

4. Ensemble d'électrodes selon la revendication 2 ou 3, dans lequel la fractalisation locale comprend une structuration du contour de l'électrode, de sorte que des rapports localement différents entre un diamètre d'électrode et la surface de l'électrode sont établis.

5. Ensemble d'électrodes selon la revendication 4, dans lequel le contour de l'électrode présente une géométrie en forme d'étoile ou de flocon de neige.

6. Ensemble d'électrodes selon l'une quelconque des revendications 2 à 5, dans lequel la fractalisation locale comprend une interruption de la couche électriquement conductrice agissant vers l'extérieur.

7. Ensemble d'électrodes selon la revendication 6, dans lequel l'interruption est formée en recouvrant partiellement la couche électriquement conductrice (102) d'une couche électriquement isolante (110).

8. Ensemble d'électrodes selon la revendication 6 ou 7, dans lequel l'interruption est formée en intégrant des pistes électriquement conductrices (120) dans la structure de support électriquement isolante (104).

9. Ensemble d'électrodes selon l'une quelconque des revendications 2 à 8, dans lequel une couche d'agent adhésif (122) est disposée entre la structure de support électriquement isolante (104) et la couche électriquement conductrice (102), et la fractalisation locale comprend une structuration de la couche d'agent adhésif (122), de sorte que l'adhérence est répartie de manière variée sur la surface de l'électrode.

10. Ensemble d'électrodes selon l'une quelconque des revendications 2 à 9, dans lequel la fractalisation locale comprend une variation de l'épaisseur de la couche de film mince (102) répartie sur la surface de l'électrode.

11. Ensemble d'électrodes selon la revendication 10, dans lequel la surface de l'électrode est localement renforcée par une pluralité de protubérances (128) réparties sur la surface de l'électrode.

12. Ensemble d'électrodes selon l'une quelconque des revendications 2 à 11, dans lequel la fractalisation locale comprend une variation, répartie sur la surface de l'électrode, d'une densité des défauts dans le matériau électriquement conducteur de l'électrode.

13. Ensemble d'électrodes selon l'une quelconque des revendications 2 à 12, dans lequel la structure de support électriquement isolante (104) comprend un matériau polymère.

14. Ensemble d'électrodes selon l'une quelconque des revendications 2 à 13, dans lequel la couche de film mince (102) comprend du platine, de l'iridium, et/ou de l'oxyde d'iridium, et/ou il existe un revêtement d'électrode constitué de métaux, d'oxydes métalliques, de nitrures métalliques ou de couches de carbone.
